# FASCICULE DE BREVET EUROPEEN

(11) **EP 3 946 170 B1**
(45) Date de publication et mention de la délivrance du brevet: **22.02.2023**
(21) Numéro de dépôt: 20712593.1
(22) Date de dépôt: 25.03.2020
(51) Int. Cl.: A61F 2/78, A61F 2/50

(54) **GAINE EN MATERIAU ÉLASTOMÈRE POUR UN MANCHON DE PROTHÈSE, ET MANCHON SUR MESURE POUR UNE PROTHÈSE**
SHEATH AUS ELASTOMERISCHEM MATERIAL FÜR EINE PROSTHESEHÜLLE UND BENUTZERDEFINIERTE HÜLLE FÜR EINE PROSTHESE
SHEATH OF ELASTOMERIC MATERIAL FOR A PROSTHESIS SLEEVE AND CUSTOM SLEEVE FOR A PROSTHESIS

(30) Priorité: 02.04.2019 FR 1903491
(43) Date de publication de la demande: 09.02.2022
(73) Titulaire: Paillet, Stéphane, 26230 Valaurie (FR)
(72) Inventeur: PAILLET, Stéphane, 84820 Visan (FR)
(74) Mandataire: IP Trust
(86) Numéro de dépôt international: PCT/EP2020/058347
(87) Numéro de publication internationale: WO 2020/200956

(56) Documents cités:
- EP-A1- 2 674 134
- EP-A2- 2 076 222
- DE-U1-202005 010 949
- FR-A1- 2 799 953
- FR-A1- 2 994 079
- US-A1- 2004 260 403

## Description

### DOMAINE TECHNIQUE DE L'INVENTION

La présente invention concerne une gaine en matériau élastomère pour un manchon de prothèse, ainsi qu'un manchon sur mesure pour une prothèse.

### ETAT DE LA TECHNIQUE

Lorsqu'un individu ou sujet ayant subi une amputation d'une extrémité d'un membre doit être équipé d'une prothèse, il est habituel de placer un manchon entre le moignon et la prothèse. Ledit manchon sert d'interface cutanée entre la prothèse et le membre, visant à améliorer la tenue de la prothèse sur la peau et à améliorer le confort du sujet.

Le manchon doit être spécialement adapté à la taille et à la forme du moignon du sujet.

Une première possibilité est de sélectionner, parmi une gamme de manchons standards existant dans différentes tailles, celui qui est le plus adapté au sujet, à partir de mesures prises sur le sujet.

Cependant, il est rare, souvent difficile, de pouvoir trouver parmi ces manchons standards un manchon qui convienne parfaitement au sujet.

Pour pallier cet inconvénient, il est connu de fabriquer le manchon sur mesure, à partir de dimensions prises sur le sujet ou à partir d'un moulage du membre (moignon) à appareiller.

Pour minimiser le temps et le coût de confection d'un tel manchon, des procédés consistent à utiliser une préforme fabriquée au préalable, pouvant se présenter en plusieurs tailles standardisées parmi lesquelles on peut choisir la plus adaptée au sujet, et de façonner ladite préforme afin de l'adapter au membre du sujet.

Ainsi, par exemple, le document FR 2 799 953 décrit un procédé de fabrication d'un manchon comprenant la fourniture d'une préforme thermoformable, notamment en une mousse de polyoléfine ou en éthylène vinyl acétate (EVA), le chauffage de ladite préforme pour la ramollir, puis l'enfilage de la préforme directement sur le moignon du sujet ou sur un moule définissant la forme extérieure dudit moignon.

Le document FR 2 994 079 décrit quant à lui un procédé de fabrication d'un manchon comprenant la fourniture d'une préforme thermoformable, notamment en un copolymère styrène-éthylène-butylène-styrène (SEBS), réalisée par injection plastique et présente une épaisseur uniforme, la mise en place de ladite préforme sur un moule définissant une réduction du moignon, le chauffage de l'ensemble préforme/moule et le démoulage après refroidissement.

Cependant, la conformation des manchons obtenus par ces procédés est limitée.

En effet, la préforme se fige après conformage à chaud, en particulier après son enfilage sur le moignon du sujet. Dès lors, le profil de la préforme correspond à celui du moignon du sujet à l'instant où la préforme est assemblée sur le moignon. Lorsque l'individu est amené à se mouvoir, le profil du moignon peut être modifié en fonction du mouvement. Le profil du manchon n'est alors plus adapté à celui du moignon, ce qui est inconfortable pour le sujet.

### BREVE DESCRIPTION DE L'INVENTION

Un but de l'invention est de surmonter les inconvénients précédents.

L'invention vise notamment à proposer un manchon de prothèse qui s'adapte au profil du moignon du sujet à appareiller.

A cette fin, l'invention propose une gaine en matériau élastomère pour un manchon de prothèse, comprenant une paroi interne et une paroi externe délimitant un corps de gaine qui s'étend entre une extrémité proximale adaptée pour enfiler le corps de gaine sur un moignon et une extrémité distale. La gaine est principalement caractérisée en ce qu'elle est munie à son extrémité distale d'une cavité conformable ménagée dans le corps de gaine qui est ouverte sur l'extérieur de la gaine via une ouverture ménagée dans la paroi externe.

L'invention se rapporte ainsi à une gaine en matériau élastomère pour un manchon de prothèse, comprenant une paroi interne et une paroi externe délimitant un corps de gaine qui s'étend entre une extrémité proximale adaptée pour enfiler le corps de gaine sur un moignon et une extrémité distale, la gaine étant caractérisée en ce qu'elle est munie à son extrémité distale d'une cavité conformable ménagée dans le corps de gaine qui est ouverte sur l'extérieur via une ouverture prévue dans la paroi externe de la gaine.

De manière optionnelle, la gaine peut comprendre une ou plusieurs des caractéristiques suivantes :
- le matériau élastomère de la gaine comprend un gel de silicone, un élastomère thermoplastique, un polyuréthane, ou un polyuréthane contenant du fluor ;
- l'épaisseur du corps de gaine entre la paroi interne et la cavité est inférieure à l'épaisseur du corps de gaine entre la paroi externe et la cavité.

L'invention se rapporte également à un procédé de fabrication d'une gaine pour un manchon de prothèse telle que décrite précédemment, comprenant les étapes suivantes :
- fourniture d'un ensemble de moulage, comprenant un moule de fabrication et un contre-moule formant une chambre de moulage,
- mise en place d'une pièce d'empreinte dans la chambre de moulage en regard de l'extrémité distale du moule de fabrication,
- injection d'un matériau élastomère dans la chambre de moulage pour former la gaine,
- retrait de la pièce d'empreinte de l'ensemble de moulage via une ouverture ménagée dans la paroi externe de la gaine, afin de former une cavité dans la gaine au niveau de l'extrémité distale de la gaine, dont la forme correspond à celle de la pièce d'empreinte.

De manière optionnelle, la pièce d'empreinte peut comprendre un corps central et une pluralité d'éléments d'empreinte montés de manière amovible sur le corps central, le retrait de la pièce d'empreinte étant réalisé en retirant successivement les éléments d'empreinte du corps central par l'ouverture.

L'invention concerne aussi un manchon sur mesure pour une prothèse, comprenant une gaine en matériau élastomère comprenant une paroi interne et une paroi externe délimitant un corps de gaine qui s'étend entre une extrémité proximale adaptée pour enfiler le corps de gaine sur un moignon et une extrémité distale, le manchon étant caractérisé en ce que la gaine est munie à son extrémité distale d'une cavité ménagée dans le corps de gaine, ladite cavité étant remplie au moins partiellement d'un matériau polymère.

De manière optionnelle, le manchon peut comprendre une ou plusieurs des caractéristiques suivantes :
- le manchon comprend en outre un revêtement d'un matériau polymère enrobant la gaine ;
- le manchon comprend en outre :
   - au moins un élément choisi parmi : un renfort polymère, une couche d'un tissu anti-élongation, une cupule distale et un tissu de drainage de l'air,
   - un revêtement d'un matériau polymère réticulable enrobant la gaine et ledit au moins un élément;
- le matériau polymère qui remplit la cavité est différent du matériau élastomère de la gaine ;
- le matériau polymère qui remplit la cavité est un élastomère thermoplastique choisi parmi : un silicone, un polyuréthane, ou un polyuréthane contenant du fluor ;
- le manchon comprend en outre un moyen d'accrochage fixé à l'extrémité distale du manchon, adapté pour fixer le manchon à une prothèse.

L'invention concerne également un procédé de fabrication d'un manchon sur mesure pour une prothèse, comprenant les étapes suivantes :
- fourniture d'un moule d'un moignon d'un sujet destiné à recevoir ladite prothèse,
- fourniture d'une gaine telle que décrite précédemment,
- mise en place de la gaine sur le moule du moignon via l'extrémité proximale de la gaine,
- injection d'un matériau polymère dans la cavité via l'ouverture ménagée dans la paroi externe de la gaine, de sorte à remplir au moins partiellement la cavité et à conformer ladite cavité remplie de matériau polymère au profil du moule du moignon.

De manière optionnelle, le procédé peut comprendre une ou plusieurs des caractéristiques suivantes :
- le procédé comprend en outre les étapes suivantes :
   - mise en place d'une bâche à vide autour de la gaine et dudit au moins un élément,
   - mise sous vide et injection d'un polymère réticulable à température ambiante dans ladite bâche à vide, de sorte à former un revêtement d'épaisseur uniforme sur la gaine ;
- le procédé comprend en outre une étape d'enduction ou de drapage de la gaine à l'aide d'un polymère réticulable ;
- le procédé comprend avant la mise en place de la bâche à vide, la mise en place, sur la gaine, d'au moins un élément parmi : un renfort en polymère, une couche d'un tissu anti-élongation, une cupule distale et une gaine de drainage de l'air
- le procédé comprend en outre une étape de fixation d'un moyen d'accrochage à l'extrémité distale du manchon, adapté pour fixer le manchon à une prothèse ;
- le matériau polymère injecté dans la cavité est différent du matériau élastomère de la gaine ;
- le matériau polymère injecté dans la cavité comprend un matériau choisi parmi : un silicone, un polyuréthane, ou un polyuréthane contenant du fluor ;

### DESCRIPTION DES FIGURES

D'autres avantages et caractéristiques de l'invention apparaîtront à la lecture de la description suivante donnée à titre d'exemple illustratif et non limitatif, en référence aux figures annexées suivantes :
[Fig. 1] est une vue de côté en coupe d'un moule d'un moignon d'un sujet à équiper d'une prothèse ;
[Fig. 2] est une vue de côté en coupe de la gaine mise en place sur le moule de Fig. 1 ;
[Fig. 3] est une vue générale en perspective d'un moule de fabrication pour former la gaine selon un mode de réalisation ;
[Fig. 4] est une vue de côté du moule de fabrication de Fig. 3 ;
[Fig. 5] est une vue de côté en coupe selon l'axe A-A du moule de fabrication de Fig. 4 ;

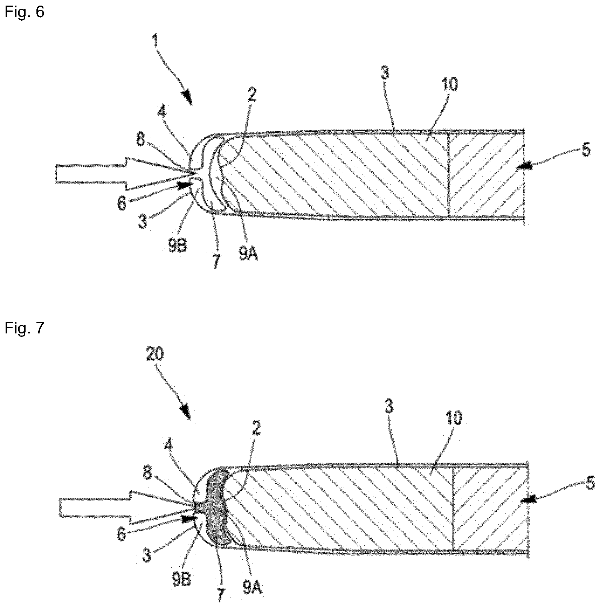

[Fig. 6] est une vue de côté en coupe de la gaine mise en place sur le moule du moignon de Fig. 1, illustrant l'injection d'un matériau polymère dans la cavité de ladite gaine ;
[Fig. 7] est une vue de côté en coupe du manchon obtenu après l'injection du matériau polymère dans la cavité de la gaine ;

### DESCRIPTION DETAILLEE DE MODES DE REALISATION DE L'INVENTION

Un premier objet de l'invention concerne une gaine en matériau élastomère pour un manchon de prothèse.

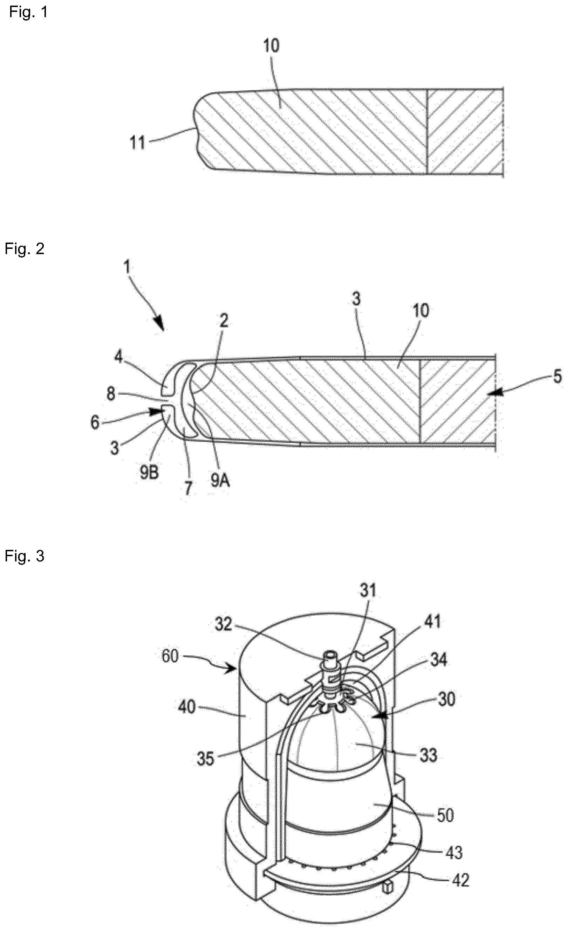

La figure 1 illustre de manière schématique un moule 10 du membre à appareiller d'un sujet, appelé moule du moignon dans le présent texte, et la figure 2 illustre un mode de réalisation d'une gaine 1 selon l'invention positionnée sur le moule du moignon pour former ultérieurement un manchon.

De manière avantageuse, le moule du moignon 10 est réduit par rapport au moignon, par application d'un taux de réduction défini par des abaques de rectification qui sont couramment utilisée dans le domaine de la conception de prothèses. L'application d'une telle réduction permet que le manchon final soit monté avec un léger serrage sur le moignon du sujet, afin d'assurer une bonne tenue du manchon.

Le moule du moignon 10 peut être fabriqué par toute technique connue, par exemple à partir d'un moulage du moignon, ou à partir d'une image tridimensionnelle dudit moignon. L'extrémité distale 11 du moule présente ainsi un profil qui correspond à celui du moignon.

Le moule 10 peut être en résine, en plâtre, en mousse de polyuréthane ou en tout autre matériau adapté à la mise en oeuvre du procédé de fabrication de la gaine.

En référence à la figure 2, la gaine 1 comprend une paroi interne 2 et une paroi externe 3, qui sont sensiblement en regard l'une de l'autre, et qui délimitent ainsi un corps de gaine 4.

Le corps de gaine 4 est réalisé dans un matériau élastomère, tel qu'un gel de silicone par exemple qui convient tout particulièrement. Le matériau élastomère est de préférence un élastomère silicone réticulable à température ambiante, appelé « RTV silicone » en anglais (Room Température Vulcanization), qui est formé par mélange de deux composants, en présence d'un catalyseur, qui réticulent idéalement à une température comprise entre 20 et 25°C. Le matériau élastomère peut également être un élastomère silicone réticulable à chaud, appelé « HTV silicone » en anglais (High Température Vulcanization). Le matériau élastomère de la gaine peut être également un élastomère thermoplastique, un polyuréthane, ou un polyuréthane contenant du fluor.

Le corps de gaine 4 s'étend entre une extrémité proximale 5 et une extrémité distale 6. L'extrémité 5 dite « proximale » correspond à l'extrémité par laquelle le moignon du sujet est inséré dans la gaine. A cet effet, l'extrémité proximale 5 de la gaine est donc ouverte et présente une dimension adaptée au passage du moignon. L'extrémité 6 dite « distale » correspond à l'extrémité de la gaine qui est opposée à l'extrémité proximale.

La gaine 1 comprend, au niveau de son extrémité distale 6, une cavité 7 conformable ménagée dans le corps de gaine 4. La cavité 7 est délimitée par une portion proximale 9A du corps de gaine située entre la cavité 7 et l'extrémité distale 11 du moule, et par une portion distale 9B du corps de gaine située entre la cavité et l'extrémité distale de la gaine.

La cavité 7 est dite « conformable » en ce que son volume interne est susceptible de varier lors de l'injection sous pression d'un polymère dans ladite cavité, en s'étendant en direction proximale. A cette fin, la portion proximale 9A du corps de gaine présente une épaisseur inférieure à celle de la portion distale 9B du corps de gaine, afin de rendre la portion proximale du corps de gaine davantage déformable et ainsi d'améliorer la conformabilité de la cavité. Ces aspects seront décrits plus en détail dans la suite du présent texte.

La cavité 7 est ouverte sur l'extérieur via une ouverture 8 ménagée dans la paroi externe 3 du corps de la gaine au niveau de son extrémité distale 6. Cette ouverture sert de passage pour le polymère destiné à être injecté dans la cavité 7.

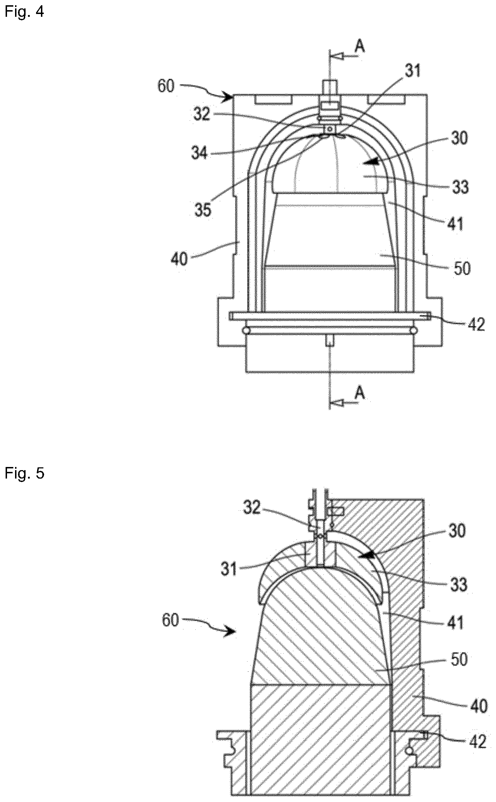

Un procédé de fabrication de la gaine 1 décrite précédemment va maintenant être décrit en référence aux figures 3 et 4.

### Fabrication de la gaine

En référence aux figures 3, 4, et 5, on commence par positionner une pièce d'empreinte 30 en regard de l'extrémité distale d'un moule de fabrication 50 d'un ensemble de moulage 60. La forme de la pièce d'empreinte correspond à celle de la cavité 7 devant être formée. Sur la figure 2, la cavité 7 présente une forme en ombrelle dont le sommet de la courbure s'étend en direction distale en s'éloignant du moule. Pour former ce type de cavité, on utilise donc une pièce d'empreinte 30 qui présente également cette forme d'ombrelle.

Selon le mode de réalisation préféré illustré sur les figures 3, 4, et 5, la pièce d'empreinte 30 comprend un corps central 31 reliée fixement à un contre-moule 40 de l'ensemble de moulage 60 par une tige 32 qui s'étend selon l'axe du moule de fabrication 50, et une pluralité d'éléments d'empreinte 33 montés de manière amovible sur le corps central 31 formant ainsi une ombrelle qui entoure l'extrémité distale 11 du moule de fabrication 50.

Le corps central 31 est muni d'une pluralité de protrusions 34 qui s'étendent en rayons depuis ledit corps central. Les éléments d'empreinte 33, sous forme de quartiers, constituent des portions de l'ombrelle, et chaque élément d'empreinte est muni d'un évidement 35 configuré pour recevoir une protrusion 34 du corps central 31 lorsque ledit élément d'empreinte 33 est monté de manière amovible sur le corps central 31.

Le contre-moule 40 est disposé autour du moule de fabrication 50 et de la pièce d'empreinte 30, de sorte à ménager une chambre de moulage 41 entre le contre-moule 40 et l'ensemble formé par le moule de fabrication 50 et la pièce d'empreinte 30.

Le contre-moule 40 comprend un socle 42 muni d'une pluralité d'évents 43. Sur la figure 4, les évents sont agencés en couronne. Ils permettent visuellement de s'assurer de l'alignement du moule de fabrication 50 avec la pièce d'empreinte 30. Ceci garantit une épaisseur uniforme de la gaine 1 formée ultérieurement.

Le matériau polymère constitutif de la gaine 1 est ensuite injecté dans la chambre de moulage 41.

Lors de l'injection, le polymère se répartit dans la chambre de moulage 41 autour du moule de fabrication 50 et de la pièce d'empreinte 30, et en particulier autour et au contact de la tige 32 de la pièce d'empreinte formant ainsi l'ouverture 8 de la gaine.

Après l'injection, on retire la pièce d'empreinte de la gaine via l'ouverture 8 afin de former la cavité 7 dans le corps de gaine 4. La forme de la cavité correspond ainsi à celle de la pièce d'empreinte 30.

Sur les figures 3, 4, et 5, la pièce d'empreinte présente une forme symétrique. Il est entendu cependant que la pièce d'empreinte peut présenter une forme asymétrique, et conduire ainsi à la formation d'une cavité elle-même asymétrique.

Selon le mode de réalisation illustré sur les figures 3, 4, et 5, le retrait de la pièce d'empreinte 30 du corps de gaine est réalisé en retirant successivement les éléments d'empreinte 33 du corps central 31 de la pièce d'empreinte par l'ouverture 8 de la gaine. Ceci permet de retirer de manière simple et rapide la pièce d'empreinte, sans endommager la gaine.

### Fabrication du manchon

A partir de la gaine obtenue précédemment, on forme le manchon final 20 en remplissant la cavité 7 avec un deuxième matériau polymère. Pour ce faire, on met en place la gaine 1 sur le moule du moignon 10 via l'extrémité proximale 5 de la gaine. On procède ensuite à l'injection sous pression du deuxième matériau polymère dans la cavité 7 via l'ouverture 8 de la gaine, de sorte à remplir au moins partiellement la cavité.

Le deuxième matériau polymère peut être identique ou différent du matériau élastomère de la gaine, et est de préférence choisi parmi : un silicone, un élastomère thermoplastique, un polyuréthane, ou un polyuréthane contenant du fluor. Le deuxième matériau polymère est choisi de manière à permettre un confort satisfaisant pour le sujet, tout en étant suffisamment robuste pour assurer une tenue mécanique suffisante et donc conserver l'intégrité structurelle du manchon.

L'injection est réalisée sous une pression comprise entre 0,01 bar et 8 bars.

Selon un premier mode de réalisation l'injection est réalisée avec une seringue. Dans ce cas, la pression d'injection est comprise entre 0,01 bar et 5 bar, ce qui est compatible avec l'utilisation d'une seringue.

Selon un deuxième mode de réalisation l'injection est réalisée à l'aide d'un pistolet mécanique, d'un pistolet électrique, ou d'un pistolet pneumatique. Dans ce cas, la pression d'injection est comprise entre 1 bar et 8 bars. Le pistolet pneumatique est muni d'une cartouche dont la pression dans la cartouche correspond à la pression d'injection du matériau polymère.

Lors de l'injection du deuxième matériau polymère dans la cavité 7, le volume interne de la cavité varie en s'étendant en direction proximale. En détail, la paroi proximale de la cavité est déplacée en direction proximale du fait de la pression exercée par le polymère. L'épaisseur de la portion proximale 9A du corps de gaine se réduit. La paroi proximale de la cavité vient alors au contact de l'extrémité distale 11 du moule du moignon et se conforme au profil du moule du moignon. Ainsi, on obtient un manchon dans lequel la cavité 7 remplie du deuxième matériau polymère est conformée au profil du moule du moignon 10.

Bien que la description qui précède ne porte que sur une cavité, il va de soi que l'on pourrait également ménager plusieurs cavités dans la gaine, en mettant en oeuvre le procédé exposé.

Un avantage de cette conception du manchon est que l'on peut utiliser des matériaux différents pour le corps de la gaine et l'insert polymérique remplissant chaque cavité, même si ces matériaux ne sont pas compatibles l'un avec l'autre, par exemple en termes d'adhésion. L'insert étant emprisonné dans la cavité, il n'est en effet pas susceptible de se désolidariser de la gaine. Le choix de matériaux adaptés est donc élargi.

Selon un mode de réalisation préféré, on positionne par ailleurs sur la gaine différents éléments destinés à être intégrés au manchon, puis on forme (par injection, par drapage ou par enduction) une couche constituée d'un troisième matériau polymère sur la gaine agencée sur le moule afin de former un revêtement autour de la gaine. Les éléments sont alors noyés dans le revêtement ainsi formé.

Ces éléments peuvent comprendre :
- un ou plusieurs renforts, qui forment des surépaisseurs sur le manchon, et qui se présentent généralement sous la forme de patches de polymère (par exemple de silicone) ;
- une ou plusieurs couches d'un tissu anti-élongation, qui est un tissu étirable dans une seule direction, en vue de permettre de contrôler l'élongation du manchon ;
- une cupule destinée à être positionnée au niveau du moignon et placée par conséquent à l'extrémité distale de la gaine, ladite cupule pouvant être pourvue ou non d'un élément de fixation de la prothèse (par exemple, un embout taraudé destiné à maintenir la prothèse par vissage),
- une gaine secondaire, par exemple, en polyamide, présentant des mailles suffisamment larges pour permettre le drainage de l'air contenu entre la gaine 1 (gaine primaire) et la gaine secondaire lors de la mise sous vide qui sera effectuée ultérieurement,
- éventuellement, un tissu élastique contribuant à renforcer le manchon et/ou à améliorer son esthétique.

Le matériau polymère du revêtement comprend avantageusement un polymère réticulable à basse température permettant de solidariser l'ensemble des éléments à la gaine, et à donner au manchon sa forme définitive.

Avant d'injecter le matériau polymère destiné à former le revêtement, ou après avoir draper la gaine de matériau polymère, une bâche à vide est mise en place autour de la préforme. La bâche à vide et les caractéristiques techniques de l'injection sont telles que décrites précédemment pour la formation de la gaine. On note que cette étape mettant en oeuvre la bâche à vide n'est pas impérative lorsque le matériau polymère est disposé par enduction, par exemple au pinceau ou au pistolet.

Le polymère destiné à former le revêtement est avantageusement du silicone ou un autre polymère réticulable à température ambiante (RTV) ou à haute température (HTV). Ce troisième polymère doit être compatible avec le matériau de la gaine, c'est-à-dire présenter de bonnes qualités d'accroche sur la gaine, afin d'assurer la cohésion du manchon.

L'épaisseur du revêtement déposé autour de la gaine est de préférence de l'ordre de 0,1 à 1 mm, mais elle peut être plus importante en fonction des éléments éventuellement placés sur la gaine. Comme cela a été indiqué précédemment, la bâche à vide permet d'assurer l'uniformité du revêtement lorsque le matériau polymère est injecté ou drapé. Ainsi, la qualité du manchon final ne dépend pas de la compétence de l'opérateur.

La durée nécessaire à la réticulation est de l'ordre de 5 à 60 minutes, mais peut varier fortement en fonction des polymères utilisés.

Ainsi, aucun chauffage n'est nécessaire pour former le revêtement.

Cependant, il peut être avantageux d'utiliser une étuve pour accélérer le processus de réticulation. De plus, lorsque le troisième matériau polymère est un silicone, une cuisson de celui-ci est vivement conseillée. Cette étape de traitement thermique, lorsqu'elle est réalisée, est conduite après 'enlèvement de la bâche à vide.

On note qu'il peut également être avantageux de former une couche de revêtement autour de la gaine à l'aide du troisième matériau polymère même lorsqu'aucun des éléments cités ci-dessus n'est intégré au manchon.

Une fois le polymère réticulé, l'opérateur retire la bâche à vide et procède le cas échéant à la finition du manchon.

Ladite finition peut consister en le collage d'un tissu à base d'élasthanne et/ou en l'application d'une peinture autoglissante. En l'absence d'un tel revêtement favorisant le glissement, on pourra appliquer du talc sur le manchon pour favoriser le glissement du revêtement sur lui-même pendant la mise en place du manchon sur le moignon.

Le manchon 20 selon l'invention s'adapte parfaitement à tout profil du moignon, grâce au fait que la conformation de la gaine 1 au profil du moignon est réalisée sur mesure sur le moule du moignon 10 correspondant.

On peut avantageusement fixer un moyen d'accrochage à l'extrémité distale du manchon ainsi obtenu. Ce moyen de fixation permet de fixer le manchon à une prothèse.

## Revendications

1. Gaine (1) en matériau élastomère pour un manchon de prothèse, comprenant une paroi interne (2) et une paroi externe (3) délimitant un corps de gaine (4) qui s'étend entre une extrémité proximale (5) adaptée pour enfiler le corps de gaine (4) sur un moignon et une extrémité distale (6), la gaine (1) étant **caractérisée en ce qu'**elle est munie à son extrémité distale (6) d'une cavité (7) conformable ménagée dans le corps de gaine (4) qui est ouverte sur l'extérieur via une ouverture (8) prévue dans la paroi externe (3) de la gaine.

2. Gaine (1) selon la revendication 1, dans laquelle le matériau élastomère de la gaine (1) comprend un gel de silicone, un élastomère thermoplastique, un polyuréthane, ou un polyuréthane contenant du fluor.

3. Gaine (1) selon la revendication 1 ou la revendication 2, dans laquelle l'épaisseur du corps de gaine (4) entre la paroi interne (2) et la cavité (7) est inférieure à l'épaisseur du corps de gaine (4) entre la paroi externe (3) et la cavité (7).

4. Procédé de fabrication d'une gaine (1) pour un manchon de prothèse selon l'une quelconque des revendications précédentes, comprenant les étapes suivantes :
- fourniture d'un ensemble de moulage (60), comprenant un moule de fabrication (50) et un contre-moule (40) formant une chambre de moulage (41),
- mise en place d'une pièce d'empreinte (30) dans la chambre de moulage (41) en regard de l'extrémité distale du moule de fabrication (50),
- injection d'un matériau élastomère dans la chambre de moulage (41) pour former la gaine (1),
- retrait de la pièce d'empreinte (30) de l'ensemble de moulage (60) via une ouverture (8) ménagée dans la paroi externe (3) de la gaine, afin de former une cavité (7) dans la gaine (1) au niveau de l'extrémité distale de la gaine, dont la forme correspond à celle de la pièce d'empreinte (30).

5. Procédé selon la revendication 4, dans lequel la pièce d'empreinte (30) comprend un corps central (31) et une pluralité d'éléments d'empreinte (33) montés de manière amovible sur le corps central, le retrait de la pièce d'empreinte (30) étant réalisé en retirant successivement les éléments d'empreinte (33) du corps central (31) par l'ouverture (8).

6. Manchon (20) sur mesure pour une prothèse, comprenant une gaine (1) en matériau élastomère comprenant une paroi interne (2) et une paroi externe (3) délimitant un corps de gaine (4) qui s'étend entre une extrémité proximale (5) adaptée pour enfiler le corps de gaine (4) sur un moignon et une extrémité distale (6), le manchon (20) étant **caractérisé en ce que** la gaine (1) est munie à son extrémité distale (6) d'une cavité (7) ménagée dans le corps de gaine (4), ladite cavité (7) étant remplie au moins partiellement d'un matériau polymère.

7. Manchon (20) selon la revendication 6, comprenant en outre un revêtement d'un matériau polymère enrobant la gaine (1).

8. : Manchon (20) selon la revendication 6, comprenant en outre
- au moins un élément choisi parmi : un renfort polymère, une couche d'un tissu anti-élongation, une cupule distale et un tissu de drainage de l'air,
- un revêtement d'un matériau polymère réticulable enrobant la gaine (1) et ledit au moins un élément.

9. Manchon (20) selon l'une des revendications 6 à 8, dans lequel le matériau polymère qui remplit la cavité (7) est différent du matériau élastomère de la gaine (1).

10. Manchon (20) selon l'une quelconque des revendications 6 à 9, dans lequel le matériau polymère qui remplit la cavité (7) est un élastomère thermoplastique choisi parmi : un silicone, un polyuréthane, ou un polyuréthane contenant du fluor.

11. Manchon (20) selon l'une des revendications 6 à 10, comprenant en outre un moyen d'accrochage fixé à l'extrémité distale du manchon, adapté pour fixer le manchon à une prothèse.

12. Procédé de fabrication d'un manchon (20) sur mesure pour une prothèse, comprenant les étapes suivantes :
- fourniture d'un moule (10) d'un moignon d'un sujet destiné à recevoir ladite prothèse,
- fourniture d'une gaine (1) selon l'une des revendications 1 à 3,
- mise en place de la gaine (1) sur le moule (10) du moignon via l'extrémité proximale (5) de la gaine,
- injection d'un matériau polymère dans la cavité (7) via l'ouverture (8) ménagée dans la paroi externe (3) de la gaine, de sorte à remplir au moins partiellement la cavité (7) et à conformer ladite cavité remplie de matériau polymère au profil du moule (10) du moignon.

13. Procédé selon la revendication 12, comprenant en outre les étapes suivantes : ,
- mise en place d'une bâche à vide autour de la gaine (1) et dudit au moins un élément,
- mise sous vide et injection d'un polymère réticulable à température ambiante dans ladite bâche à vide, de sorte à former un revêtement d'épaisseur uniforme sur la gaine (1).

14. Procédé selon la revendication 12, comprenant une étape d'enduction ou de drapage de la gaine à l'aide d'un polymère réticulable.

15. Procédé selon la revendication 13 ou 14, comprenant avant la mise en place de la bâche à vide, la mise en place, sur la gaine (1), d'au moins un élément parmi : un renfort en polymère, une couche d'un tissu anti-élongation, une cupule distale et une gaine de drainage de l'air.

16. Procédé selon l'une des revendications 12 à 15, comprenant en outre une étape de fixation d'un moyen d'accrochage (31) à l'extrémité distale du manchon, adapté pour fixer le manchon à une prothèse.

17. Procédé selon l'une quelconque des revendications 12 à 16, dans lequel le matériau polymère injecté dans la cavité (7) est différent du matériau élastomère de la gaine (1).

18. Procédé selon l'une quelconque des revendications 12 à 17, dans lequel le matériau polymère injecté dans la cavité (7) comprend un matériau choisi parmi : un silicone, un polyuréthane, ou un polyuréthane contenant du fluor.

## Patentansprüche

1. Hülle (1) aus elastomerem Material für einen Prothesenliner, umfassend eine Innenwand (2) und eine Außenwand (3), die einen Hüllenkörper (4) begrenzen, der sich zwischen einem proximalen Ende (5), das zum Aufziehen des Hüllenkörpers (4) auf einen Stumpf geeignet ist, und einem distalen Ende (6) erstreckt, wobei die Hülle (1) **dadurch gekennzeichnet ist, dass** sie an ihrem distalen Ende (6) mit einem anpassungsfähigen Hohlraum (7) versehen ist, der in dem Hüllenkörper (4) ausgebildet ist und der über eine Öffnung (8), die in der Außenwand (3) der Hülle vorgesehen ist, nach außen hin offen ist.

2. Hülle (1) nach Anspruch 1, wobei das elastomere Material der Hülle (1) ein Silikongel, ein thermoplastisches Elastomer, ein Polyurethan oder ein fluorhaltiges Polyurethan umfasst.

3. Hülle (1) nach Anspruch 1 oder 2, wobei die Dicke des Hüllenkörpers (4) zwischen der Innenwand (2) und dem Hohlraum (7) geringer ist als die Dicke des Hüllenkörpers (4) zwischen der Außenwand (3) und dem Hohlraum (7).

4. Verfahren zum Herstellen einer Hülle (1) für einen Prothesenliner nach einem der vorstehenden Ansprüche, umfassend die folgenden Schritte:
- Bereitstellen einer Formanordnung (60), umfassend eine Herstellungsform (50) und eine Gegenform (40), die eine Formkammer (41) bilden,
- Platzieren eines Abdruckstücks (30) in der Formkammer (41) gegenüber dem distalen Ende der Herstellungsform (50),
- Einspritzen eines elastomeren Materials in die Formkammer (41), um die Hülle (1) zu bilden,
- Entfernen des Abdruckstücks (30) aus der Formanordnung (60) durch eine Öffnung (8), die in der Außenwand (3) der Hülle ausgebildet ist, um einen Hohlraum (7) in der Hülle (1) am distalen Ende der Hülle zu bilden, dessen Form der des Abdruckstücks (30) entspricht.

5. Verfahren nach Anspruch 4, wobei das Abdruckstück (30) einen zentralen Körper (31) und mehrere Abdruckelemente (33) umfasst, die abnehmbar an dem zentralen Körper angebracht sind, wobei das Entfernen des Abdruckstücks (30) durch aufeinanderfolgendes Entfernen der Abdruckelemente (33) von dem zentralen Körper (31) durch die Öffnung (8) durchgeführt wird.

6. Maßgefertigter Liner (20) für eine Prothese, umfassend eine Hülle (1) aus elastomerem Material mit einer Innenwand (2) und einer Außenwand (3), die einen Hüllenkörper (4) begrenzen, der sich zwischen einem proximalen Ende (5), das zum Aufziehen des Hüllenkörpers (4) auf einen Stumpf geeignet ist, und einem distalen Ende (6) erstreckt, wobei der Liner (20) **dadurch gekennzeichnet ist, dass** die Hülle (1) an ihrem distalen Ende (6) mit einem Hohlraum (7) versehen ist, der in dem Hüllenkörper (4) ausgebildet ist, wobei der Hohlraum (7) zumindest teilweise mit einem polymeren Material gefüllt ist.

7. Liner (20) nach Anspruch 6, umfassend ferner eine Beschichtung aus einem Polymermaterial, das die Hülle (1) umhüllt.

8. Liner (20) nach Anspruch 6, ferner umfassend
- mindestens eines der folgenden Elemente: eine Polymerverstärkung, eine Schicht aus einem Anti-Dehnungsgewebe, eine distale Pfanne und ein luftabführendes Gewebe,
- eine Beschichtung aus einem vernetzbaren Polymermaterial, welche die Hülle (1) und das mindestens eine Element umhüllt.

9. Liner (20) nach einem der Ansprüche 6 bis 8, wobei das Polymermaterial, das den Hohlraum (7) füllt, von dem elastomeren Material der Hülle (1) verschieden ist.

10. Liner (20) nach einem der Ansprüche 6 bis 9, wobei das Polymermaterial, das den Hohlraum (7) ausfüllt, ein thermoplastisches Elastomer ist, das ausgewählt ist aus: Silikon, Polyurethan oder fluorhaltigem Polyurethan.

11. Liner (20) nach einem der Ansprüche 6 bis 10, ferner umfassend ein am distalen Ende des Liners befestigtes Einhakmittel, das dazu geeignet ist, den Liner an einer Prothese zu befestigen.

12. Verfahren zum Herstellen eines maßgefertigten Liners (20) für eine Prothese, umfassend die folgenden Schritte:
- Bereitstellen einer Form (10) eines Stumpfes eines Patienten, der die Prothese tragen soll,
- Bereitstellen einer Hülle (1) nach einem der Ansprüche 1 bis 3,
- Anbringen der Hülle (1) an der Form (10) des Stumpfes über das proximale Ende (5) der Hülle,
- Einspritzen eines Polymermaterials in den Hohlraum (7) durch die Öffnung (8), die in der Außenwand (3) der Hülle ausgebildet ist, um den Hohlraum (7) zumindest teilweise zu füllen und den mit Polymermaterial gefüllten Hohlraum an das Profil der Form (10) des Stumpfes anzupassen.

13. Verfahren nach Anspruch 12, ferner umfassend die folgenden Schritte: ,
- Anbringen eines Vakuumbehälters um die Hülle (1) und das mindestens eine Element,
- Vakuumieren und Einspritzen eines bei Raumtemperatur vernetzbaren Polymers in den Vakuumbehälter, so dass auf der Hülle (1) eine Beschichtung von gleichmäßiger Dicke gebildet wird.

14. Verfahren nach Anspruch 12, umfassend einen Schritt des Beschichtens oder Umhüllens der Hülle mit einem vernetzbaren Polymer.

15. Verfahren nach Anspruch 13 oder 14, umfassend vor dem Anbringen des Vakuumbehälters das Anbringen von mindestens einem der folgenden Elemente auf der Hülle (1): einer Polymerverstärkung, einer Schicht aus einem Anti-Dehnungsgewebe, einer distalen Pfanne und einer luftabführenden Hülle.

16. Verfahren nach einem der Ansprüche 12 bis 15, ferner umfassend einen Schritt des Befestigens eines Einhakmittels (31) am distalen Ende des Liners, das dazu geeignet ist, den Liner an einer Prothese zu befestigen.

17. Verfahren nach einem der Ansprüche 12 bis 16, wobei das in den Hohlraum (7) eingespritzte Polymermaterial von dem elastomeren Material der Hülle (1) verschieden ist.

18. Verfahren nach einem der Ansprüche 12 bis 17, wobei das in den Hohlraum (7) eingespritzte Polymermaterial ein Material umfasst, das ausgewählt ist aus: einem Silikon, einem Polyurethan oder einem fluorhaltigen Polyurethan.

## Claims

1. Sheath (1) of elastomeric material for a prosthesis liner, comprising an inner wall (2) and an outer wall (3) delimiting a sheath body (4) that extends between a proximal end (5) suitable to slip the sheath body (4) onto a stump and a distal end (6), the sheath (1) being **characterized in that** it is provided at its distal end (6) with a conformable cavity (7) that is formed in the sheath body (4), which cavity is open to the outside via an opening (8) provided in the outer wall (3) of the sheath.

2. Sheath (1) according to claim 1, wherein the elastomeric material of the sheath (1) comprises a silicone gel, a thermoplastic elastomer, a polyurethane, or a polyurethane containing fluorine.

3. Sheath (1) according to claim 1 or claim 2, wherein the thickness of the sheath body (4) between the inner wall (2) and the cavity (7) is less than the thickness of the sheath body (4) between the outer wall (3) and the cavity (7).

4. Method of manufacturing a sheath (1) for a prosthesis liner according to any one of the preceding claims, comprising the following steps:
- provision of a molding assembly (60), comprising a manufacturing mold (50) and a counter-mold (40) forming a molding chamber (41),
- placement of an impression piece (30) in the molding chamber (41) facing the distal end of the manufacturing mold (50),
- injection of an elastomeric material into the molding chamber (41) to form the sheath (1),
- removal of the impression piece (30) from the molding assembly (60) via an opening (8) in the outer wall (3) of the sheath, to form a cavity (7) in the sheath (1) at the distal end of the sheath, the shape of which corresponds to that of the impression piece (30).

5. Method according to claim 4, wherein the impression piece (30) comprises a central body (31) and a plurality of impression elements (33) that are removably mounted on the central body, the removal of the impression piece (30) being accomplished by successively removing the impression elements (33) from the central body (31) through the opening (8).

6. Custom-made liner (20) for a prosthesis, comprising a sheath (1) made from an elastomeric material comprising an inner wall (2) and an outer wall (3) delimiting a sheath body (4) that extends between a proximal end (5) suitable to slip the sheath body (4) onto a stump and a distal end (6), the liner (20) being **characterized in that** the sheath (1) is provided at its distal end (6) with a cavity (7) that is formed in the sheath body (4), which cavity (7) being filled at least partially with a polymer material.

7. The liner (20) according to claim 6, further comprising a coating of a polymer material encasing the sheath (1).

8. Liner (20) according to claim 6, further comprising at least
- one element chosen from: a polymer reinforcement, a layer of an anti-elongation fabric, a distal cup and an air drainage fabric,
- a coating of a cross-linkable polymer material encasing the sheath (1) and the said at least one element.

9. Liner (20) according to any one of claims 6 to 8, wherein the polymer material that fills the cavity (7) is different from the elastomeric material of the sheath (1).

10. Liner (20) according to any one of claims 6 to 9, wherein the polymer material that fills the cavity (7) is a thermoplastic elastomer chosen from: a silicone, a polyurethane, or a polyurethane containing fluorine.

11. Liner (20) according to any one of claims 6 to 10, further comprising a fastening means attached to the distal end of the liner, suitable for attaching the liner to a prosthesis.

12. Method of manufacturing a custom-made liner (20) for a prosthesis, comprising the following steps:
- provision of a mold (10) of a stump of a subject intended to receive said prosthesis,
- provision of a sheath (1) according to any one of claims 1 to 3,
- placement of the sheath (1) on the mold (10) of the stump via the proximal end (5) of the sheath,
- injection of a polymer material into the cavity (7) via the opening (8) made in the outer wall (3) of the sheath, so as to at least partially fill the cavity (7) and to conform said cavity filled with polymer material to the profile of the mold (10) of the stump.

13. Method according to claim 12, further comprising the following steps:
- placement of a vacuum bagging film around the sheath (1) and the said at least one element,
- placement under vacuum and injection of a polymer that can be cross-linked at room temperature into the said vacuum bagging film, so as to form a coating of uniform thickness on the sheath (1).

14. Method according to claim 12, comprising a step of coating or draping of the sheath using a cross-linkable polymer.

15. Method according to claim 13 or claim 14, comprising, prior to the placement of the vacuum bagging film, the positioning, on the sheath (1), of at least one of: a polymer reinforcement, a layer of an anti-elongation fabric, a distal cup and an air drainage sheath.

16. Method according to any one of claims 12 to 15, further comprising a step of attaching a fastening means (31) to the distal end of the liner, suitable for attaching the liner to a prosthesis.

17. Method according to any one of claims 12 to 16, wherein the polymer material injected into the cavity (7) is different from the elastomeric material of the sheath (1).

18. Method according to any one of claims 12 to 17, wherein the polymer material injected into the cavity (7) comprises a material chosen from: a silicone, a polyurethane, or a polyurethane containing fluorine.
